(19) Europäisches Patentamt
European Patent Office
Office européen des brevets

(11) Veröffentlichungsnummer: **0 150 748**
**B1**

(12) # EUROPÄISCHE PATENTSCHRIFT

(45) Veröffentlichungstag der Patentschrift: **31.10.90**

(51) Int. Cl.[5]: **C 07 D 333/38**, A 01 N 43/10, A 01 N 47/04

(21) Anmeldenummer: **85100293.1**

(22) Anmeldetag: **14.01.85**

(54) **Acyloxythiophen-Derivate.**

(30) Priorität: **26.01.84 DE 3402625**

(43) Veröffentlichungstag der Anmeldung:
**07.08.85 Patentblatt 85/32**

(45) Bekanntmachung des Hinweises auf die Patenterteilung:
**31.10.90 Patentblatt 90/44**

(84) Benannte Vertragsstaaten:
**AT BE CH DE FR GB IT LI NL**

(56) Entgegenhaltungen:
**EP-A-0 093 384**

(73) Patentinhaber: **BAYER AG**
**D-5090 Leverkusen 1 Bayerwerk (DE)**

(72) Erfinder: **Daum, Werner, Dr.**
**Bärenstrasse 18**
**D-4150 Krefeld (DE)**
Erfinder: **Klauke, Erich, Dr.**
**Eichendorffweg 8**
**D-5068 Odenthal (DE)**
Erfinder: **Kühle, Engelbert, Dr.**
**von-Bodelschwingh-Strasse 42**
**D-5060 Bergisch-Gladbach (DE)**
Erfinder: **Brandes, Wilhelm, Dr.**
**Eichendorffstrasse 3**
**D-5653 Leichlingen 1 (DE)**
Erfinder: **Reinecke, Paul, Dr.**
**Lessingstrasse 11**
**D-5090 Leverkusen 3 (DE)**

**Die Akte enthält technische Angaben, die nach dem Eingang der Anmeldung eingereicht wurden und die nicht in dieser Patentschrift enthalten sind.**

EP 0 150 748 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue Acyloxythiophen-Derivate, ein Verfahren zu ihrer Herstellung und ihre Verwendung als Pflanzenschutzmittel, vor allem als Fungizide.

Es ist bereits bekannt, daß Thiophen-Derivate, wie beispielsweise das 2,5-Bis-(butoxycarbonyl)-3-methyl-4-styrylcarbonyloxy-thiophen und das 2,5-Bis-ethoxycarbonyl-3,4-bis-benzoyloxy-thiophen, fungizide Eigenschaften besitzen (vgl. EP 93 384 und EP 32 748).

Die Wirkung dieser Verbindungen kann unter bestimmten Umständen, z.B. bei niedrigen Aufwandmengen und -konzentrationen, nicht immer ganz befriedigend sein.

Es wurden neue Acyloxythiophen-Derivate der allgemeinen Formel (I)

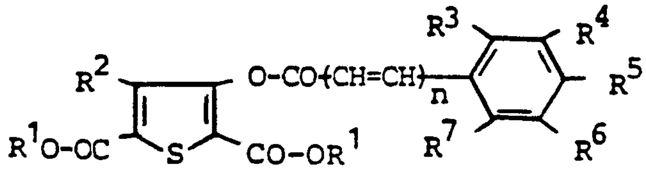

(I)

in welcher

$R^1$ für Alkyl mit 1 bis 5 Kohlenstoffatomen, für Alkoxyalkyl oder für Alkylthioalkyl mit 1 bis 5 Kohlenstoffatomen je Alkylteil, für Halogenalkyl mit 1 oder 2 Kohlenstoffatomen und bis zu 5 gleichen oder verschiedenen Halogenatomen, wie Fluor oder Chlor, für Cyanalkyl mit 1 bis 5 Kohlenstoffatomen im Alkylteil, für Trialkylsilylmethyl mit 1 bis 3 Kohlenstoffatomen je Alkylteil, für Alkenyl mit 2 bis 4 Kohlenstoffatomen oder für Alkinyl mit 3 bis 5 Kohlenstoffatomen steht,

$R^2$ für Alkyl mit 1 bis 4 Kohlenstoffatomen steht,

n für 0 oder 1 steht,

$R^3$ bis $R^5$ gleich oder verschieden sind und für Wasserstoff, für Alkyl oder Alkoxy mit 1 bis 4 Kohlenstoffatomen, für Halogenalkyl mit 1 oder 2 Kohlenstoffatomen und bis zu 5 gleichen oder verschiedenen Halogenatomen, wie Fluor oder Chlor, für Halogenalkoxy oder Halogenalkylthio oder Halogenalkylsulfinyl oder Halogenalkylsulfonyl mit 1 oder 2 Kohlenstoffatomen und bis zu 5 gleichen oder verschiedenen Halogenatomen, wie Fluor oder Chlor, je Halogenalkylrest, für N-Halogenalkyl-N-halogenalkylthioamino mit 1 oder 2 Kohlenstoffatomen und bis zu 5 gleichen oder verschiedenen Halogenatomen, wie Fluor oder Chlor, je Halogenalkylrest, für Chlor oder Fluor stehen und

$R^6$ und $R^7$ für Wasserstoff, Fluor, Chlor oder Trifluormethyl stehen, mit der Maßgabe, daß einer der Reste $R^3$ bis $R^7$ ein fluorhaltiger Rest ist oder mindestens 3 der Reste $R^3$ bis $R^7$ für Fluor und/oder Chlor stehen, gefunden.

Weiterhin wurde gefunden, daß man die neuen Acyloxythiophen-Derivate der allgemeinen Formel (I)

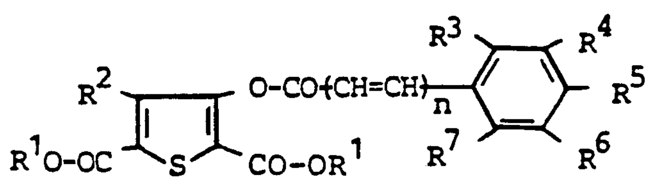

(I)

in welcher

$R^1$ für Alkyl mit 1 bis 5 Kohlenstoffatomen, für Alkoxyalkyl oder für Alkylthioalkyl mit 1 bis 5 Kohlenstoffatomen je Alkylteil, für Halogenalkyl mit 1 oder 2 Kohlenstoffatomen, und bis zu 5 gleichen oder verschiedenen Halogenatomen, wie Fluor oder Chlor, für Cyanalkyl mit 1 bis 5 Kohlenstoffatomen im Alkylteil, für Trialkylsilylmethyl mit 1 bis 3 Kohlenstoffatomen je Alkylteil, für Alkenyl mit 2 bis 4 Kohlenstoffatomen oder für Alkinyl mit 3 bis 5 Kohlenstoffatomen steht,

$R^2$ für Alkyl mit 1 bis 4 Kohlenstoffatomen steht,

n für 0 oder 1 steht,

$R^3$ bis $R^5$ gleich oder verschieden sind und für Wasserstoff, für Alkyl oder Alkoxy mit 1 bis 4 Kohlenstoffatomen, für Halogenalkyl mit 1 oder 2 Kohlenstoffatomen und bis zu 5 gleichen oder verschiedenen Halogenatomen, wie Fluor oder Chlor, für Halogenalkoxy oder Halogenalkylthio oder Halogenalkylsulfinyl oder Halogenalkylsulfonyl mit 1 oder 2 Kohlenstoffatomen und bis zu 5 gleichen oder verschiedenen Halogenatomen, wie Fluor oder Chlor, je Halogenalkylrest, für N-Halogenalkyl-N-halogenalkylthioamino mit 1 oder 2 Kohlenstoffatomen und bis zu 5 gleichen oder verschiedenen Halogenatomen, wie Fluor oder Chlor, je Halogenalkylrest, für Chlor oder Fluor stehen und

$R^6$ und $R^7$ für Wasserstoff, Fluor, Chlor oder Trifluormethyl stehen, mit der Maßgabe, daß einer der Reste $R^3$ bis $R^7$ ein fluorhaltiger Rest ist oder mindestens 3 der Reste $R^3$ bis $R^7$ für Fluor und/oder Chlor stehen,

erhält, wenn man ein Carbonsäurederivat der allgemeinen Formel (II)

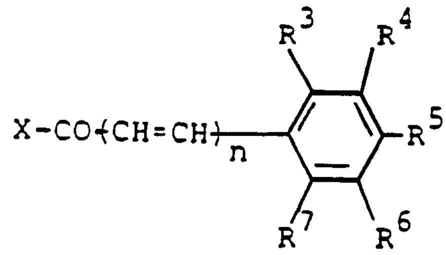

(II)

in welcher

X für Halogen, Hydroxy oder den Rest

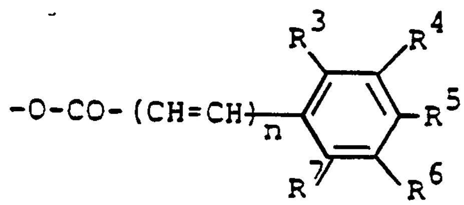

steht, worin in den Formeln n, $R^3$ bis $R^7$ die oben angegebene Bedeutung haben, mit 3-Hydroxy-thiophen-Derivaten der allgemeinen Formel (III)

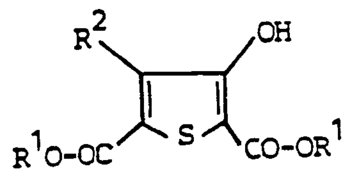

(III)

in welcher

$R^1$ und $R^2$ die oben angegebene Bedeutung haben, gegebenenfalls in Gegenwart eines Lösungs- oder Verdünnungsmittels und gegebenenfalls in Gegenwart eines Säurebinders bzw. wasserabspaltenden Mittels, umsetzt.

Weiterhin wurde gefunden, daß die neuen Acyloxythiophen-Derivate der Formel (I) fungizide Eigenschaften besitzen.

Überraschenderweise zeigen die erfindungsgemäßen Acyloxythiophen-Derivate der Formel (I) eine bessere fungizide Wirksamkeit als die aus dem Stand der Technik bekannten Fungizide, wie z.B. 2,5 - Bis - (isopropoxy - carbonyl) - 3 - methyl - 4 - (3 - methylbenzoyloxy) - thiophen, 2 - sec. - Butyl - 4,6 - dinitrophenyl - 3 - methylcrotonat und/oder 6-Methyl-2-oxo-1,3-dithiolo-[4,5-b]-chinoxalin.

Die erfindungsgemäßen Acyloxythiophen-Derivate sind durch die Formel (I) allgemein definiert. Bevorzugt sind Verbindungen der Formel (I), bei welchen

$R^1$ für Methyl, Ethyl, n- oder iso-Propyl, 2,2-Dimethyl-propyl, Methoxymethyl, 2-Methoxyethyl, Ethoxymethyl, 2-Ethoxyethyl, Methylthiomethyl, 2-Methylthio-ethyl, Ethylthiomethyl, 2-Ethylthio-ethyl, 2,2,2-Trifluoroethyl, Cyanmethyl, 2-Cyan-ethyl, Trimethylsilylmethyl, Allyl, Methallyl, 3-Propinyl oder 1,1-Dimethyl-3-propinyl steht,

$R^2$ für Methyl, Ethyl, n-Propyl, iso-Propyl, n-Butyl, sec.-Butyl, iso-Butyl oder tert.-Butyl steht,

n für 0 oder 1 steht,

$R^3$ bis $R^5$ gleich oder verschieden sind und für Wasserstoff, Isopropyl, Methoxy, Tetrafluorethoxy, Trifluorchlorethoxy, Trifluormethyl, 2,2,2-Trifluorethyl, Trifluormethoxy, Trifluormethylthio, Trifluormethylsulfinyl, Trifluormethylsulfonyl, Chlordifluormethoxy, Chlordifluormethylthio, Chlordifluormethylsulfinyl, Chlordifluormethylsulfonyl, N-Trifluormethyl-N-dichlorfluormethylthio-amino-, Chlor oder Fluor stehen und

$R^6$ und $R^7$ für Wasserstoff oder Fluor stehen, mit der Maßgabe, daß einer der Reste $R^3$ bis $R^7$ ein fluorhaltiger Rest ist oder mindestens 3 der Reste $R^3$ oder $R^7$ für Fluor und/oder Chlor stehen.

Insbesondere seien Verbindungen der Formel (I) genannt, in denen

$R^1$ für Methyl, Ethyl, iso-Propyl, 2,2-Dimethylpropyl, Trifluorethyl oder Trimethylsilylmethyl, steht,

$R^2$ für Methyl, Ethyl, iso-Propyl oder tert.-Butyl steht und

$R^3$ für Wasserstoff, Fluor, Trifluormethyl, N-Trifluormethyl-N-dichlorfluormethylthio-amino steht,

$R^4$ für Wasserstoff, Trifluormethyl, Trifluormethoxy, N-Trifluormethyl-N-dichlorfluormethylthio-amino, Methyl, Fluor oder Chlor steht,

$R^5$ für Wasserstoff, iso-Propyl, Methoxy, Trifluormethyl, Trifluormethoxy, Trifluormethylthio, Trifluormethylsulfinyl, Trifluormethylsulfonyl, Chlordifluormethoxy, Chlordifluormethylthio, N-Trifluormethyl-N-dichlorfluormethylthio-amino, Fluor oder Chlor steht,

$R^6$ und $R^7$ für Wasserstoff oder Fluor und

n für 0 und 1 stehen.

Verwendet man als Ausgangsverbindungen zur Herstellung der erfindungsgemäßen Verbindungen der Formel (I) beispielsweise 2,5-Bis-(trimethylsilylmethyloxycarbonyl)-3-hydroxy-4-ethyl-thiophen und 3-Trifluormethyl-4-isopropylbenzoesäure und Dicyclohexylcarbodiimid als wasser-abspaltendes Agens, so kann der Reaktionsverlauf durch das folgende Reaktionsschema wiedergegeben werden:

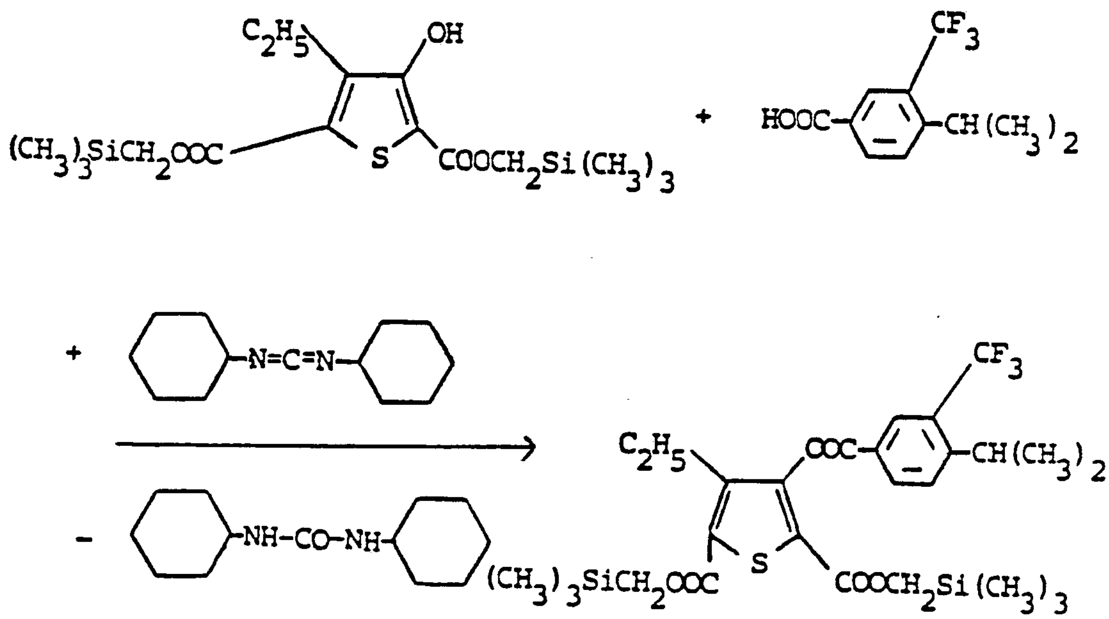

Die als Ausgangsstoffe zur Durchführung des erfindungsgemäßen Verfahrens benötigten Carbonsäurederivate sind durch die Formel (II) allgemein definiert. Die Verbindungen sind überwiegend bekannt und können nach bekannten Verfahren hergestellt werden.

So können einige der Verbindungen z.B. hergestellt werden aus Fluorcarbonyl-N-(trifluormethylsulfenyl)-anilinen und Ameisensäure im Temperaturbereich zwischen 50°C und 100°C (vgl. DE—OS—1 810 580 bzw. die entsprechende GB—PS—1 229 083, DAS—1 293 754 bzw. FR—PS—1 524 722).

Zu nennen sind hier insbesondere:

2-, 3- und 4-Trifluormethylzimtsäure-, Pentafluorbenzoesäure-, 2-, 3- und 4-Trifluormethylbenzoesäure-, 2-, 3- und 4-(N-Trifluormethyl-N-dichlorfluormethylsulfenylamino)-benzoesäure-, 3-(N-Trifluormethyl-N-dichlorfluormethylsulfenyl)-4-methyl-benzoesäure-, 3-(N-Trifluormethyl-N-dichlorfluormethylsulfenyl)-4-methoxybenzoesäure-, 3-Methyl-4-(N-trifluormethyl-N-dichlorfluormethylsulfenyl)-benzoesäure-, 2,6-Di-trifluormethylbenzoesäure-, 3,5-Di-trifluormethylbenzoesäure-, 2-Methoxy-6-trifluormethylbenzoesäure-, 2,3,4,5-Tetrafluorbenzoesäure-, 3,4,6-Trifluorbenzoesäure-, 3-Chlor-4,5,6-Trifluorbenzoesäure-, 2,4,6-Trifluor-3,5-dichlorbenzoesäure-, 3,5-Dichlor-4-fluorbenzoesäure-, 3- und 4-Trifluormethoxybenzoesäure-, 3-Trifluormethyl-4-isopropylbenzoesäure-, 3-Chlor-4-trifluormethoxy-benzoesäure-, 3-Chlor-4-trifluormethoxy-benzoesäure-, 4-Chlordifluormethoxy- und 4-Chlordifluormethylthiobenzoesäure- sowie 4-Trifluormethylsulfinyl und 4-Trifluormethylsulfonylbenzoesäurehalogenid und -anhydrid sowie die entsprechenden freien Säuren.

Weiterhin werden für die Umsetzungen zu den erfindungsgemäßen Verbindungen 3-Hydroxythiophen-Derivate benötigt, die durch die Formel (III) allgemein definiert sind. Die Ausgangsverbindungen der Formel (III) sind teilweise bekannt, können aber nach generell bekannten Verfahren hergestellt werden, so z.B. aus Thiodiessigsäureestern und 2-Oxocarbonsäureestern unter alkalischen Bedingungen, z.B. unter der Einwirkung von Kalium-tert.-butylat, und anschließend an die Kondensation wird mit einer Säure behandelt (vgl. EP—93 384 und DAS—1020641). Die Reaktion kann durch das folgende Schema veranschlaulicht werden:

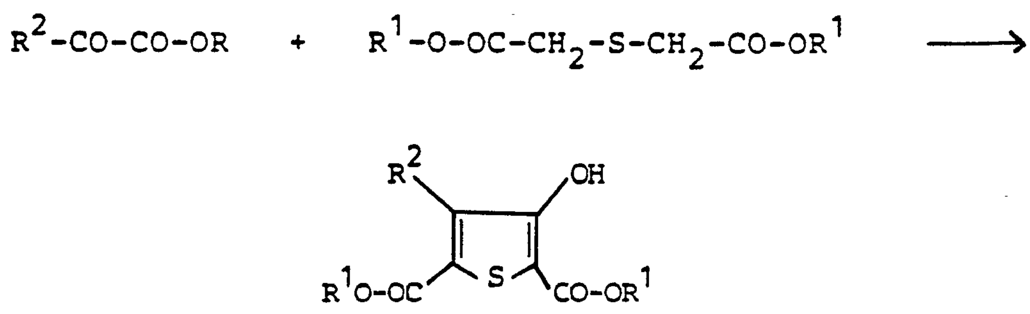

(III)

Verbindungen der Formel (IIIa)

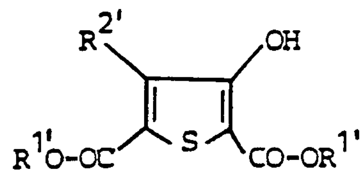

(IIIa)

in welcher

$R^{1'}$ für Methyl oder 2,2-Dimethylpropyl steht und

$R^{2'}$ für Methyl oder iso-Propyl steht, sind neu, können aber nach den oben angegebenen Verfahren hergestellt werden.

Zu nennen sind im einzelnen:

3-Hydroxy-4-methyl-thiophen-2,5-dicarbonsäure-methyl-, -ethyl-, -isopropyl-, -2,2-dimethylpropyl-, -cyanmethyl-, 2-cyanethyl-, -1-cyan-1-methyl-ethyl-, -2,2,2-trifluorethyl-, -2-methoxyethyl-, -2-butylthioethyl-, 2-ethyl-thioethyl-, trimethylsilylmethyl-, -allyl-, -methylallyl-, -propargyl- und -1,1-dimethylpropargylester; 3-Hydroxy-4-ethyl-, -4-propyl-, -4-isopropyl-, -4-butyl- und -4-tert.-butyl-thiophen-2,5-dicarbonsäure-2,2,2-trifluorethylester.

Als Verdünnungsmittel kommen für das Verfahren alle gegenüber den Reaktionspartnern inerten organischen Lösungsmittel in Frage; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Die Reaktionen können auch in heterogenen Systemen, bestehend aus Wasser und einem mit Wasser nicht mischbaren Lösungsmittel, durchgeführt werden.

Als Säurebinder für die Umsetzung werden organische Basen verwendet, vorzugsweise tertiäre Amine. Zu nennen sind hier: Chinolin, Dimethylbenzylamin, Dimethylanilin, Ethyldicyclohexylamin, Ethyldiisopropylamin, Picolin, Pyridin und Triethylamin.

Als wasserbindende Agentien beim Einsatz von Carbonsäuren (Formel II, x = OH) werden bevorzugt Carbodiimide, wie z.B. Dicyclohexylcarbodiimid, verwendet.

Die Reaktionstemperaturen und die Reaktionsdauer werden durch die Aktivität der Ausgangsprodukte bestimmt. Im allgemeinen arbeitet man etwa zwischen −50 und +80°C, vorzugsweise zwischen −10 und +60°C.

Auch kann man zur Durchführung des erfindungsgemäßen Verfahrens Alkali- oder Erdalkalisalze des umzusetzenden Thiophenderivats in einem inerten Lösungsmittel vorlegen, oder man erzeugt das Salz, indem man in eine Mischung aus dem Thiophenderivat und einem hochsiedenden Lösungsmittel Alkalilauge, Alkoholate oder eine entsprechende Erdalkaliverbindung gibt und dann vorsichtig entwässert bzw. den Alkohol abdestilliert oder man gibt ein Alkali- oder Erdalkalihydrid hinzu.

Wird die Kondensation des Hydroxythiophenderivats mit einer fluorierten Carbonsäure durch Wasserentzug mit einem Carbodiimid, beispielsweise Dicyclohexylcarbodiimid, durchgeführt, so läß sich der entstehende schwerlösliche Harnstoff meist einfach von den leichtlöslichen erfindungsgemäßen Verbindungen abtrennen.

Je nach Arbeitsbedingungen fallen die erfindungsgemäßen Wirkstoffe kristallin aus oder sie blieben im organischen Lösungsmittel gelöst und können dann nach Auswaschen der Lösung mit Wasser durch vorsichtiges Einengen der Lösung oder durch Zugabe wenig polarer organischer Lösungsmittel, wie Cyclohexan, Dibutylether oder Tetrachlorkohlenstoff, abgeschieden werden. Gegebenenfalls müssen mit Wasser mischbare polare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie auch durch Zugabe von Wasser ausgefällt werden.

Die erfindungsgemäßen Verbindungen zersetzen sich zum Teil bei höherer Temperatur; in diesen Fällen können die Schmelzpunkte nur mit geringer Genauigkeit oder überhaupt nicht ermittelt werden. Das

Vorliegen bestimmter Strukturelemente ist aus den NMR-Spektren ersichtlich.

Die erfindungsgemäßen Wirkstoffe weisen eine starke mikrobizide Wirkung auf und können zur Bekämpfung von unerwünschten Mikroorganismen praktisch eingesetzt werden.

Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden z.B. im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die aufgezählten Oberbegriffe fallen, genannt:

Botyrtis-Arten, wie beispielsweise Botrytis cinerea;
Plasmopara-Arten, wie beispielsweise Plasmopara viticola;
Uromyces-Arten, wie beispielsweise Uromyces appendiculatus;
Spaerotheca-Arten, wie beispielsweise Spaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder avenae;
Septoria-Arten, wie beispielsweise Septoria nodorum;
Tilletia-Arten, wie beispielsweise Tilletia caries;
Xanthomonas-Arten, wie beispielsweise Xanthomonas oryzae;
Pseudomonas-Arten, wie beispielsweise Xanthomonas lachrymans;
Pyricularia-Arten, wie beispielsweise Pyricularia oryzae;
Pellicularia-Arten, wie beispielsweise Pellicularia sasakii;
Pyrenophora-Arten, wie beispielsweise Pyrenophora teres (Konidienform: Drechslera, Syn: Helminthosporium);
Leptosphaeria-Arten, wie beispielsweise Leptosphaeria nodorum;
Cochliobolus-Arten, wie beispielsweise Cochliobolus sativus (Konidienform: Drechslera, Syn: Helminthosporium) und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigments, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie andere Fungizide, Insektizide, Akarizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

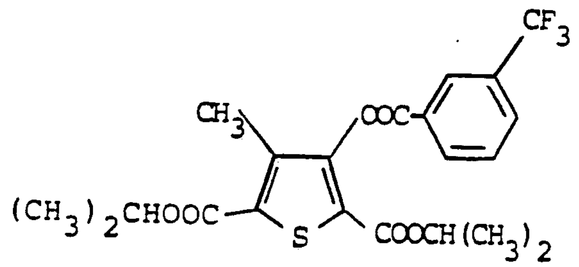

11,5 g 2,5-Bis-(isopropoxycarbonyl)-3-hydroxy-4-methyl-thiophen, 6 g Triethylamin und 100 ml wasserfreies Acetonitril werden bei 0°C vorgelegt. In 15 min werden 8,3 g 3-Trifluormethylbenzoylfluorid zugetropft. Die Reaktionsmischung wird sodann 2 h auf 58°C gehalten. Es wird mit 400 ml Ethylacetat verdünnt, mit Wasser und mit Natriumbicarbonat-Lösung gewaschen und über Natriumsulfat getrocknet. Nach Eindampfen der Lösung und Erhitzen des Rückstandes im Hochvakuum auf 60°C erhält man 16,4 g 2,5-Bis-(isopropoxycarbonyl)-3-(3-trifluormethylbenzoyloxy)-4-methyl-thiophen mit dem Brechungsindex: $n_D^{20}$: 1,5101.

NMR 80 MHz $CDCl_3$ $CH(CH_3)_2$ 12 p, 2 d = 1,17 + 1,4 ppm
$CH(CH_3)_2$ 2 p, m = 4,95—5,48 ppm
$CH_3$-Thiophen 3 p, s = 2,42 ppm
Benzoyl-H 2 p, m = 7,5—7,7 ppm
2 p, m = 8,08—8,33 ppm.

Auf die gleiche Weise können die Verbindungen der Formel (I) hergestellt werden:

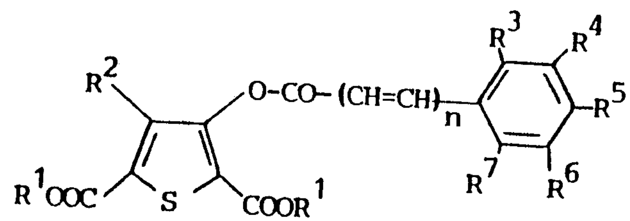

| Beispiel-Nr. | $R^1$ | $R^2$ | n | $R^3$ | $R^4$ | $R^5$ | $R^6$ | $R^7$ | Physikal. Daten (Schmelzpkt: °C) |
|---|---|---|---|---|---|---|---|---|---|
| 2 | $(CH_3)_2CH—$ | $CH_3$ | 0 | $CF_3$ | H | H | H | H | 78 (aus Petrolether) |
| 3 | $(CH_3)_2CH—$ | $CH_3$ | 0 | H | $OCF_3$ | H | H | H | 86 (aus Petrolether) |
| 4 | $(CH_3)_2CH—$ | $CH_3$ | 0 | H | 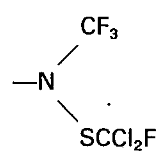 | H | H | H | viskose Masse |
| 5 | $(CH_3)_2CH—$ | $CH_3$ | 1 | $CF_3$ | H | H | H | H | 78 (aus Petrolether) |
| 6 | $CH_3$ | $CH_3$ | 0 | H | $CF_3$ | H | H | H | 136 (aus Diisopropyl-ether) |
| 7 | $CH_3$ | $CH_3$ | 0 | H | $OCF_3$ | H | H | H | 105 |

Fortsetzung

Auf die gleiche Weise können die Verbindungen der Formel (I) hergestellt werden:

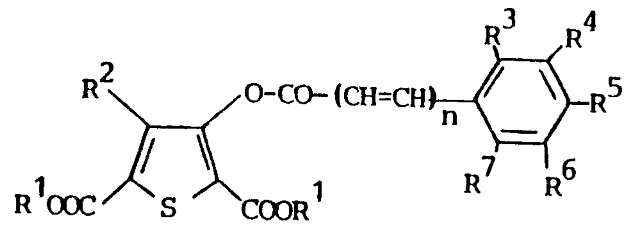

| Beispiel-Nr. | $R^1$ | $R^2$ | n | $R^3$ | $R^4$ | $R^5$ | $R^6$ | $R^7$ | Physikal. Daten (Schmelzpkt: °C) |
|---|---|---|---|---|---|---|---|---|---|
| 8 | $(CH_3)_2CH-$ | $CH_3$ | 0 | $-N(CF_3)-SCCl_2F$ | H | H | H | H | viskose Masse |
| 9 | $(CH_3)_2CH-$ | $CH_3$ | 0 | H | H | $-N(CF_3)-SCCl_2F$ | H | H | zähviskose Masse |
| 10 | $CH_3$ | $CH_3$ | 0 | H | H | $-N(CF_3)SCCl_2F$ | H | H | 88 (aus Petrolether) |
| 11 | $CH_3$ | $CH_3$ | 0 | H | $-N(CF_3)-SCCl_2F$ | H | H | H | 78 (aus Petrolether) |
| 12 | $CH_3$ | $CH_3$ | 0 | $-N(CF_3)-SCCl_2F$ | H | H | H | H | 145 (aus Petrolether) |
| 13 | $(CH_3)_2CH-$ | $CH_3$ | 0 | H | $CF_3$ | $-CH(CH_3)_2$ | H | H | viskose Masse |
| 14 | $(CH_3)_3C-CH_2-$ | $CH_3$ | 0 | H | $CF_3$ | H | H | H | viskose Masse |
| 15 | $CH_3$ | $(CH_3)_2CH-$ | 0 | H | $CF_3$ | H | H | H | 123 (aus Diisopropylether) |

Die Ausgangsverbindungen der Formel (II) können z.B. folgendermaßen hergestellt werden:

Beispiel a:

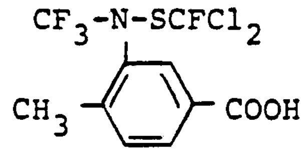

10 g 3-N-(Fluordichlormethylmercapto)-N-trifluormethylamino-4-methylbenzoesäurefluorid werden in 50 ml Ameisensäure gelöst und 2 Stunden auf 90—100°C erhitzt. Hierbei entwickelt sich fortlaufend Gas (CO, HF, HCOF). Nach dem Erkalten kristallisiert die entsprechende Säure (9 g) aus, die nach dem Umkristallisieren aus Acetonitril bei 154—156°C schmilzt.

In analoger Weise erhält man aus den zugehörigen Säurefluoriden die folgenden Benzoesäuren:

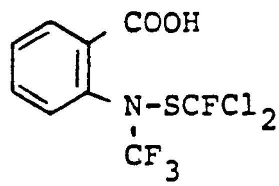

F 123°C

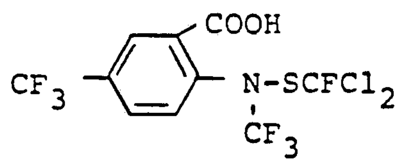

F 150°C

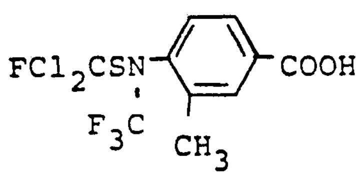

F 155°C

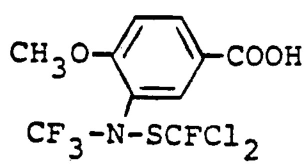

F 183°C

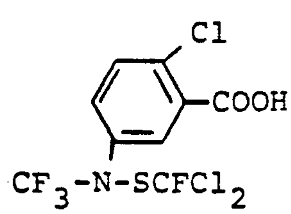

F 81-85°C

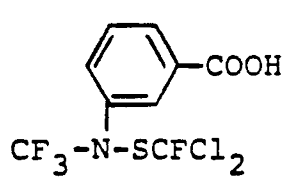

F 76-80°C

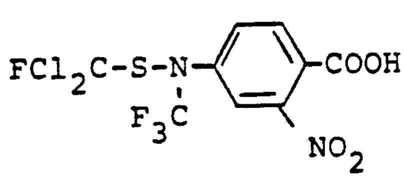

F 80-82°C

Beispiel b:

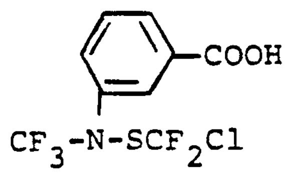

32,5 g 3-N-(Difluorchlormethylmercapto)-N-trifluormethylaminobenzoesäurefluorid werden in 70 ml Ameisensäure 2 Stunden lang erhitzt. In der Kälte kristallisieren 17 g der zugehörigen Carbonsäure vom Schmelzpunkt 80—82°C aus.

Beispiel c:

Die Ausgangsverbindungen der Formel (III) können nach den Vorschriften, die in EP 93 384 und DAS 10 20 641 beschrieben sind, hergestellt werden. Die folgenden Verbindungen sind neu:

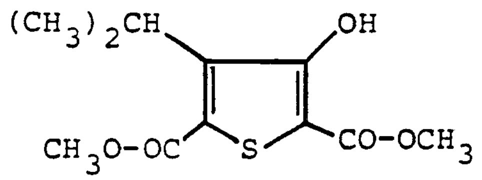

Schmelzpunkt: 123,5°C
(aus Diethylketon)

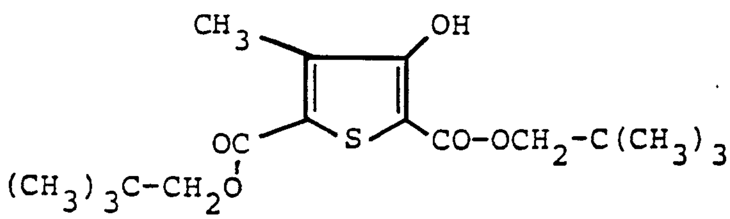

Schmelzpunkt: 75°C
(aus Acetonitril)

Beispiel A

Sphaerotheca-Test (Gurke)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75% im Gewächshaus aufgestellt.

10 Tage nach der Inokulation erfolgt die Auswertung.

Eine deutliche Überlegenheit in der Wirksamkeit gegenüber dem Stand der Technik zeigt in diesem Test z.B. der erfindungsgemäße Verbindung: 1.

Beispiel B

Podosphaera-Test (Apfel)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nacn Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70% aufgestellt.

9 Tage nach der Inokulation erfolgt die Auswertung.

Eine deutliche Überlegenheit in der Wirksamkeit gegenüber dem Stand der Technik zeigen in diesem Test die erfindungsgemäßen Verbindungen: 1, 3, 2 und 5.

Beispiel C

Pyrenophora teres-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1, 3, 2, 5 und 4.

**Patentansprüche**

1. Acyloxythiophen-Derivate der allgemeinen Formel (I)

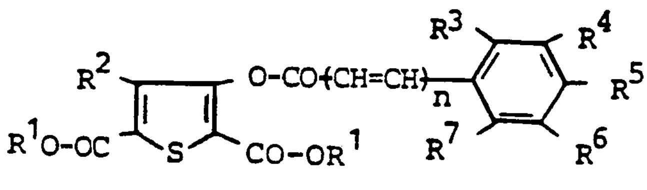

(I)

in welcher

$R^1$ für Alkyl mit 1 bis 5 Kohlenstoffatomen, für Alkoxyalkyl oder für Alkylthioalkyl mit 1 bis 5 Kohlenstoffatomen je Alkylteil, für Halogenalkyl mit 1 oder 2 Kohlenstoffatomen, und bis zu 5 gleichen oder verschiedenen Halogenatomen, für Cyanalkyl mit 1 bis 5 Kohlenstoffatomen im Alkylteil, für Trialkylsilylmethyl mit 1 bis 3 Kohlenstoffatomen je Alkylteil, für Alkenyl mit 2 bis 4 Kohlenstoffatomen oder für Alkinyl mit 3 bis 5 Kohlenstoffatomen steht,

$R^2$ für Alkyl mit 1 bis 4 Kohlenstoffatomen steht,

n für 0 oder 1 steht,

$R^3$ bis $R^5$ gleich oder verschieden sind und für Wasserstoff, für Alkyl oder Alkoxy mit 1 bis 4 Kohlenstoffatomen, für Halogenalkyl mit 1 oder 2 Kohlenstoffatomen und bis zu 5 gleichen oder verschiedenen Halogenatomen, für Halogenalkoxy oder Halogenalkylthio oder Halogenalkylsulfinyl oder Halogenalkylsulfonyl mit 1 oder 2 Kohlenstoffatomen und bis zu 5 gleichen oder verschiedenen Halogenatomen je Halogenalkylrest, für N-Halogenalkyl-N-halogenalkylthio-amino mit 1 oder 2 Kohlenstoffatomen und bis zu 5 gleichen oder verschiedenen Halogenatomen je Halogenalkylrest, für Chlor oder Fluor stehen und

$R^6$ und $R^7$ für Wasserstoff, Fluor, Chlor oder Trifluormethyl stehen, mit der Maßgabe, daß einer der Reste $R^3$ bis $R^7$ ein fluorhaltiger Rest ist oder mindestens 3 der Reste $R^3$ bis $R^7$ für Fluor und/oder Chlor stehen.

2. Acyloxythiophen-Derivate gemäß dem Anspruch 1, wobei in der Formel (I)

$R^1$ für Methyl, Ethyl, n- oder iso-Propyl, 2,2-Dimethyl-propyl, Methoxymethyl, 2-Methoxy-ethyl, Ethoxymethyl, 2-Ethoxy-ethyl, Methylthiomethyl, 2-Methylthio-ethyl, Ethylthiomethyl, 2-Ethyl-thio-ethyl, 2,2,2-Trifluor-ethyl, Cyanmethyl, 2-Cyan-ethyl, Trimethylsilylmethyl, Allyl, Methallyl, 3-Propinyl oder 1,1-Dimethyl-3-propinyl steht,

$R^2$ für Methyl, Ethyl, n-Propyl, iso-Propyl, n-Butyl, sec.-Butyl, iso-Butyl oder tert.-Butyl steht,

n für 0 oder 1 steht,

$R^3$ bis $R^5$ gleich oder verschieden sind und für Wasserstoff, Methyl, Isopropyl, Methoxy, Tetrafluorethoxy, Trifluorchlorethoxy, Trifluormethyl, 2,2,2-Trifluorethyl, Triflormethoxy, Trifluormethylthio, Trifluormethylsulfinyl, Trifluormethylsulfonyl, Chlordifluormethoxy, Chlordifluormethylthio, Chlordifluormethylsulfinyl, Chlordifluormethylsulfonyl, N-Trifluormethyl-N-dichlorfluormethylthio-amino-, Chlor oder Fluor stehen und

$R^6$ und $R^7$ für Wasserstoff oder Fluor stehen, mit der Maßgabe, daß einer der Reste fluorhaltiger Rest ist oder mindestens 3 der Reste $R^3$ oder $R^7$ für Fluor und/oder Chlor stehen.

3. Acyloxythiophen-Derivate gemäß dem Anspruch 1, wobei in der Formel (I)

$R^1$ für Methyl, Ethyl, iso-Propyl, 2,2-Dimethylpropyl, Trifluorethyl oder Trimethylsilylmethyl steht,

$R^2$ für Methyl, Ethyl, iso-Propyl oder tert.-Butyl steht und

$R^3$ für Wasserstoff, Fluor, Trifluormethyl, N-Trifluormethyl-N-dichlorfluormethylthio-amino steht,

$R^7$ für Wasserstoff, Trifluormethyl, Trifluormethoxy, N-Trifluormethyl-N-dichlorfluormethylthio-amino, Chlor, Methyl oder Fluor steht,

$R^5$ für Wasserstoff, iso-Propyl, Methoxy, Trifluormethyl, Trifluormethoxy, Trifluormethylthio, Trifluormethylsulfinyl, Trifluormethylsulfonyl, Chlordifluormethoxy, Chlordifluormethylthio oder N-Trifluormethyl-N-dichlorfluormethylthioamino steht,

$R^6$ und $R^7$ für Wasserstoff oder Fluor und

n für 0 oder 1 stehen, mit der Maßgabe, daß einer der Reste $R^3$ bis $R^7$ ein fluorhaltiger Rest ist oder mindestens 3 der Reste $R^3$ bis $R^7$ für Fluor und/oder Chlor stehen.

4. Verfahren zur Herstellung von Acyloxythiophen-Derivaten der allgemeinen Formel (I)

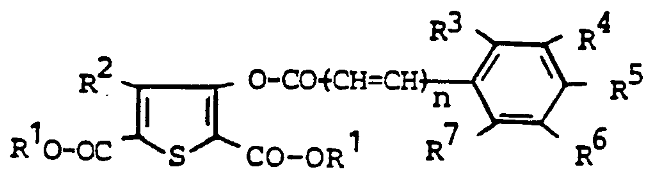

(I)

in welcher

$R^1$ für Alkyl mit 1 bis 5 Kohlenstoffatomen, für Alkoxyalkyl oder für Alkylthioalkyl mit 1 bis 5 Kohlenstoffatomen je Alkylteil, für Halogenalkyl mit 1 oder 2 Kohlenstoffatomen, und bis zu 5 gleichen oder verschiedenen Halogenatomen, für Cyanalkyl mit 1 bis 5 Kohlenstoffatomen im Alkylteil, für Trialkylsilylmethyl mit 1 bis 3 Kohlenstoffatomen je Alkylteil, für Alkenyl mit 2 bis 4 Kohlenstoffatomen oder für Alkinyl mit 3 bis 5 Kohlenstoffatomen steht,

$R^2$ für Alkyl mit 1 bis 4 Kohlenstoffatomen steht,

n für 0 oder 1 steht,

$R^3$ bis $R^5$ gleich oder verschieden sind und für Wasserstoff, für Alkyl oder Alkoxy mit 1 bis 4 Kohlenstoffatomen, für Halogenalkyl mit 1 oder 2 Kohlenstoffatomen und bis zu 5 gleichen oder verschiedenen Halogenatomen, für Halogenalkoxy oder Halogenalkylthio oder Halogenalkylsulfinyl oder Halogenalkylsulfonyl mit 1 oder 2 Kohlenstoffatomen und bis zu 5 gleichen oder verschiedenen Halogenatomen je Halogenalkylrest, für N-Halogenalkyl-N-halogenalkylthio-amino mit 1 oder 2 Kohlenstoffatomen und bis zu 5 gleichen oder verschiedenen Halogenatomen je Halogenalkylrest, für Chlor oder Fluor stehen und

$R^6$ und $R^7$ für Wasserstoff, Fluor, Chlor oder Trifluormethyl stehen, mit der Maßgabe, daß einer der Reste $R^3$ bis $R^7$ ein fluorhaltiger Rest ist oder mindestens 3 der Reste $R^3$ bis $R^7$ für Fluor und/oder Chlor stehen, dadurch gekennzeichnet, daß man ein Carbonsäure-derivat der allgemeinen Formel (II)

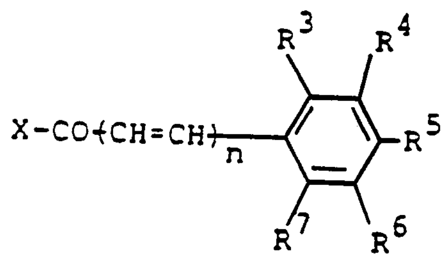

(II)

in welcher

X für Halogen, Hydroxy oder den Rest

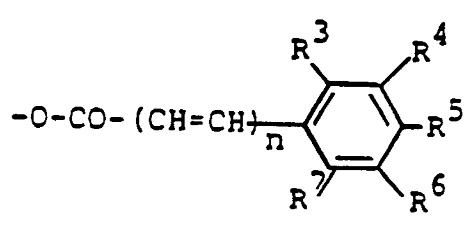

steht, worin in den Formeln n, $R^3$ bis $R^7$ die oben angegebene Bedeutung haben, mit 3-Hydroxy-thiophen-Derivaten der allgemeinen Formel (III)

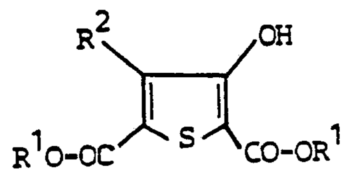

(III)

in welcher

$R^1$ und $R^2$ die oben angegebene Bedeutung haben, gegebenenfalls in Gegenwart eines Lösungs- oder Verdünnungsmittels und gegebenenfalls in Gegenwart eines Säurebinders bzw. wasserabspaltenden Mittels, umsetzt.

5. Pflanzenschutzmittel, gekennzeichnet durch einen Gehalt an mindestens einem Acyloxthiophen-Derivat der Formel (I) gemäß dem Anspruch 1.

6. Verfahren zur Bekämpfung von Pilzen und Bakterien, dadurch gekennzeichnet, daß man Acyloxythiophen-Derivate der Formel (I) gemäß dem Anspruch 1 auf Pilze oder Bakterien oder ihren Lebensraum einwirken läßt.

7. Verwendung von Acyloxythiophen-Derivaten der Formel (I) gemäß dem Anspruch 1 zur Bekämpfung von Pilzen und Bakterien.

8. Verfahren zur Herstellung von Pflanzenschutzmitteln, dadurch gekennzeichnet, daß man Acyloxythiophen-Derivate der Formel (I) gemäß dem Anspruch 1 mit Streckmitteln und/oder oberflächenaktiven Mitteln vermischt.

**Revendications**

1. Dérivés d'acyloxythiophènes de formule générale (I)

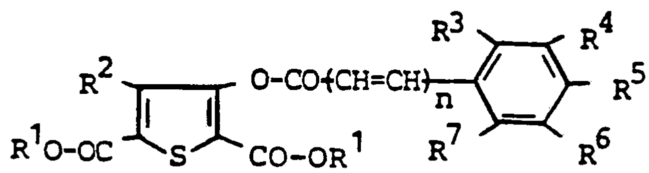

(I)

dans laquelle

$R^1$ est un groupe alkyle ayant 1 à 5 atomes de carbone, un groupe alkoxyalkyle ou un groupe alkylthioalkyle ayant 1 à 5 atomes de carbone par radical alkyle, un groupe halogénalkyle ayant 1 ou 2 atomes de carbone et jusqu'à 5 atomes d'halogènes identiques ou différents, un groupe cyanalkyle ayant 1 à 5 atomes de carbone dans le radical alkyle, un groupe trialkylsilylméthyle ayant 1 à 3 atomes de carbone par radical alkyle, un groupe alcényle de 2 à 4 atomes de carbone ou un groupe alcynyle de 3 à 5 atomes de carbone,

$R^2$ est un groupe alkyle de 1 à 4 atomes de carbone,

n a la valeur 0 ou 1,

$R^3$ à $R^5$ sont identiques ou différents et représentent l'hydrogène, un groupe alkyle ou alkoxy ayant 1 à 4 atomes de carbone, un groupe halogénalkyle ayant 1 ou 2 atomes de carbone et jusqu'à 5 atomes d'halogènes identiques ou différents, un groupe halogénalkoxy ou halogénalkylthio ou halogénalkylsulfinyle ou halogénalkylsulfonyle ayant 1 ou 2 atomes de carbone et jusqu'à 5 atomes d'halogènes identiques ou différents par reste halogénalkyle, un groupe N-halogénoalkyl-N-halogénalkylthio-amino ayant 1 ou 2 atomes de carbone et jusqu'à 5 atomes d'halogènes identiques ou différents par reste halogénalkyle, du chlore ou du fluor et

$R^6$ et $R^7$ représentent l'hydrogène, le fluor, le chlore ou un groupe trifluorométhyle, sous réserve que l'un des restes $R^3$ à $R^7$ soit un reste fluoré ou qu'au moins trois des restes $R^3$ à $R^7$ représentent du fluor et/ou du chlore.

2. Dérivés d'acyloxythiophènes suivant la revendication 1, dans la formule (I) desquels

$R^1$ est un groupe méthyle, éthyle, n-propyle, isopropyle, 2,2-diméthylpropyle, méthoxyméthyle, 2-méthoxyéthyle, éthoxyméthyle, 2-éthoxyéthyle, méthylthiométhyle, 2-méthylthioéthyle, éthylthiométhyle, 2-éthylthioéthyle, 2,2,2-trifluoréthyle, cyanométhyle, 2-cyanéthyle, triméthylsilylméthyle, allyle, méthallyle, 3-propynyle ou 1,1-diméthyl-3-propynyle,

$R^2$ est ou groupe méthyle, éthyle, n-propyle, isopropyle, n-butyle, sec.-butyle, isobutyle ou tertio-butyle,

n a la valeur 0 ou 1,

$R^3$ à $R^5$ sont identiques ou différents et représentent l'hydrogène, un groupe méthyle, isopropyle, méthoxy, tétrafluoréthoxy, trifluorochloréthoxy, trifluorométhyle, 2,2,2-trifluoréthyle, trifluorométhoxy, trifluorométhylthio, trifluorométhylsulfinyle, trifluorométhylsulfonyle, chlorodifluorométhoxy, chlorodifluorométhylthio, chlorodifluorométhylsulfinyle, chlorodifluorométhylsulfonyle, N-trifluorométhyl-N-dichlorofluorométhylthio-amino, du chlore ou du fluor et

$R^6$ et $R^7$ représentent l'hydrogène ou du fluor, sous réserve que l'un des restes soit un reste fluoré ou qu'au moins trois des restes $R^3$ à $R^7$ représente du fluor et/ou du chlore.

3. Dérivés d'acyloxythiophènes suivant la revendication 1, dans la formule (I) desquels

$R^1$ est un groupe méthyle, éthyle, isopropyle, 2,2-diméthylpropyle, trifluoréthyle ou triméthylsilylméthyle,

$R^2$ est un groupe méthyle, éthyle, isopropyle ou tertio-butyle et

$R^3$ est l'hydrogène, le fluor, un groupe trifluorométhyle, N-trifluorométhyl-N-dichlorofluorométhylthio-amino,

$R^4$ est de l'hydrogène, un groupe trifluorométhyle, trifluorométhoxy, N-trifluorométhyl-N-dichlorofluorométhylthio-amino, du chlore, un groupe méthyle ou du fluor,

$R^5$ représente de l'hydrogène, un groupe isopropyle, méthoxy, trifluorométhyle, trifluorométhoxy, trifluorométhylthio, trifluorométhylsulfinyle, trifluorométhylsulfonyle, chlorodifluorométhoxy, chlorodifluorométhylthio ou N-trifluorométhyl-N-dichlorofluorométhylthioamino,

$R^6$ et $R^7$ représentent l'hydrogène ou du fluor et

n a la valeur 0 ou 1, sous réserve que l'un des restes $R^3$ à $R^7$ soit un reste fluoré ou qu'au moins trois des restes $R^3$ à $R^7$ représente du fluor et/ou du chlore.

4. Procédé de préparation de dérivés d'acyloxythiophènes de formule générale (I)

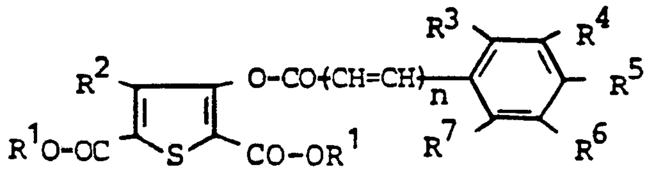

(I)

dans laquelle

$R^1$ est un groupe alkyle ayant 1 à 5 atomes de carbone, un groupe alkoxyalkyle ou un groupe alkylthioalkyle ayant 1 à 5 atomes de carbone par radical alkyle, un groupe halogénalkyle ayant 1 ou 2 atomes de carbone et jusqu'à 5 atomes d'halogènes identiques ou différents, un groupe cyanalkyle ayant 1 à 5 atomes de carbone dans le radical alkyle, un groupe trialkylsilylméthyle ayant 1 à 3 atomes de carbone par radical alkyle, un groupe alcényle de 2 à 4 atomes de carbone ou un groupe alcynyle de 3 à 5 atomes de carbone,

$R^2$ est un groupe alkyle de 1 à 4 atomes de carbone,

n a la valeur 0 ou 1,

$R^3$ à $R^5$ sont identiques ou différents et représentent l'hydrogène, un groupe alkyle ou alkoxy ayant 1 à 4 atomes de carbone, un groupe halogénalkyle ayant 1 ou 2 atomes de carbone et jusqu'à 5 atomes d'halogènes identiques ou différents, un groupe halogénalkoxy ou halogénalkylthio ou halogénalkylsulfinyle ou halogénalkylsulfonyle ayant 1 ou 2 atomes de carbone et jusqu'à 5 atomes d'halogènes identiques ou différents par reste halogénalkyle, un groupe N-halogénalkyl-N-halogénalkylthio-amino ayant 1 ou 2 atomes de carbone et jusqu'à 5 atomes d'halogènes identiques ou différents par reste halogénalkyle, du chlore ou du fluor et

$R^6$ et $R^7$ représentent l'hydrogène, le fluor, le chlore ou un groupe trifluorométhyle, sous réserve que l'un des restes $R^3$ à $R^7$ soit un reste fluoré ou qu'au moins trois des restes $R^3$ à $R^7$ représentent du fluor et/ou du chlore, caractérisé en ce qu'on fait réagir un dérivé d'acide carboxylique de formule générale (II)

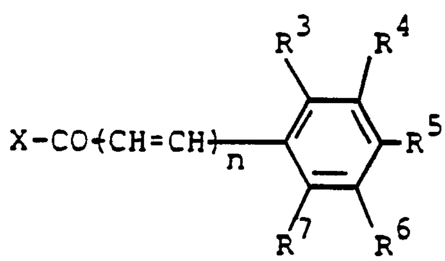

(II)

dans laquelle

X est un halogène, un groupe hydroxy ou le reste

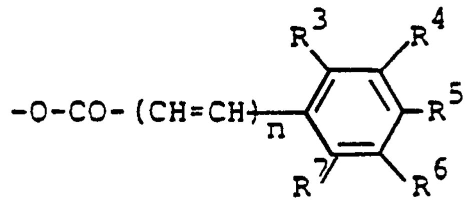

formules dans lesquelles n et $R^3$ à $R^7$ ont la définition indiquée ci-dessus, avec des dérivés de 3-hydroxythiophène de formule générale (III)

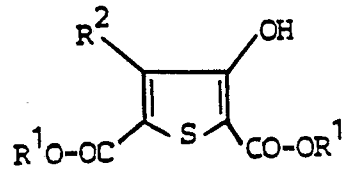

(III)

dans laquelle

$R^1$ et $R^2$ ont la définition indiquée ci-dessus, le cas échéant en présence d'un solvant ou d'un diluant et en la présence éventuelle d'un accepteur d'acide ou d'un agent déshydratant.

5. Compositions destinées à la protection des plantes, caractérisées par une teneur en au moins un dérivé d'acyloxythiophène de formule (I) suivant la revendication 1.

6. Procédé pour combattre des champignons et des bactéries, caractérisé en ce qu'on fait agir des dérivés d'acyloxythiophènes de formule (I) suivant la revendication 1 sur des champignons ou sur des bactéries ou sur leurs milieux.

7. Utilisation de dérivés d'acyloxythiophènes de formule (I) suivant la revendication 1 pour combattre des champignons ou des bactéries.

8. Procédé de préparation de compositions pour la protection des plantes, caractérisé en ce qu'on mélange des dérivés d'acyloxythiophènes de formule (I) suivant la revendication 1 avec des diluants et/ou des agents tensioactifs.

**Claims**

1. Acyloxythiophene derivatives of the general formula (I)

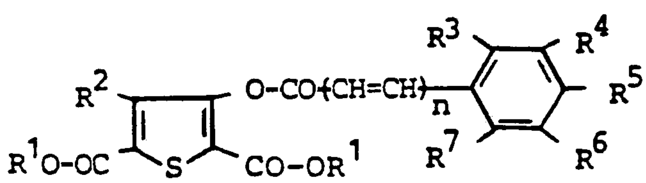

(I)

in which

$R^1$ represents alkyl with 1 to 5 carbon atoms, alkoxyalkyl or alkylthioalkyl with 1 to 5 carbon atoms per alkyl part, halogenoalkyl with 1 or 2 carbon atoms and up to 5 identical or different halogen atoms, cyanoalkyl with 1 to 5 carbon atoms in the alkyl part, trialkylsilylmethyl with 1 to 3 carbon atoms per alkyl part, alkenyl with 2 to 4 carbon atoms or alkinyl with 3 to 5 carbon atoms, $R^2$ represents alkyl with 1 to 4 carbon atoms, n represents 0 or 1,

$R^3$ to $R^5$ are identical or different and represent hydrogen, alkyl or alkoxy with 1 to 4 carbon atoms, halogenoalkyl with 1 or 2 carbon atoms and up to 5 identical or different halogen atoms, halogenoalkoxy, halogenoalkylthio, halogenoalkylsulphinyl or halogenoalkylsulphonyl with 1 or 2 carbon atoms and up to 5 identical or different halogen atoms per halogenoalkyl radical, N-halogenoalkyl-N-halogenoalkylthio-amino with 1 or 2 carbon atoms and up to 5 identical or different halogen atoms per halogenoalkyl radical, chlorine or fluorine and

$R^6$ and $R^7$ represent hydrogen, fluorine, chlorine or trifluoromethyl, with the proviso that one of the radicals $R^3$ to $R^7$ is a fluorine-containing radical or at least 3 of the radicals $R^3$ to $R^7$ represent fluorine and/or chlorine.

2. Acyloxythiophene derivatives according to Claim 1, where, in formula (I),

$R^1$ represents methyl, ethyl, n- or iso-propyl, 2,2-dimethyl-propyl, methoxymethyl, 2-methoxyethyl, ethoxymethyl, 2-ethoxyethyl, methylthiomethyl, 2-methylthio-ethyl, ethylthiomethyl, 2-ethylthio-ethyl, 2,2,2-trifluoroethyl, cyanomethyl, 2-cyano-ethyl, trimethylsilylmethyl, allyl, methallyl, 3-propinyl or 1,1-

dimethyl-3-propinyl, $R^2$ represents methyl, ethyl, n-propyl, iso-propyl, n-butyl, sec.-butyl, iso-butyl or tert.-butyl, n represents 0 or 1,

$R^3$ to $R^5$ are identical or different and represent hydrogen, methyl, isopropyl, methoxy, tetrafluoroethoxy, trifluorochloroethoxy, trifluoromethyl, 2,2,2-trifluoroethyl, trifluoromethoxy, trifluoromethylthio, trifluoromethylsulphinyl, trifluoromethylsulphonyl, chlorodifluoromethoxy, chlorodifluoromethylthio, chlorodifluoromethylsulphinyl, chlorodifluoromethylsulphonyl, N-trifluoromethyl-N-dichlorofluoromethylthio-amino, chlorine or fluorine and

$R^6$ and $R^7$ represent hydrogen or fluorine, with the proviso that one of the radicals is a fluorine-containing radical or at least 3 of the radicals $R^3$ to $R^7$ represent fluorine and/or chlorine.

3. Acyloxythiophene derivatives according to Claim 1, where, in formula (I),

$R^1$ represents methyl, ethyl, iso-propyl, 2,2-dimethylpropyl, trifluoroethyl or trimethylsilylmethyl,

$R^2$ represents methyl, ethyl, iso-propyl or tert.-butyl and

$R^3$ represents hydrogen, fluorine, triflurormethyl or N-trifluoromethyl-N-dichlorofluoromethylthioamino,

$R^4$ represents hydrogen, trifluoromethyl, trifluoromethoxy, N-trifluoromethyl-N-dichlorofluoromethyl-thio-amino, chlorine, methyl or fluorine,

$R^5$ represents hydrogen, iso-propyl, methoxy, trifluoromethyl, trifluoromethoxy, trifluoromethylthio, trifluoromethylsulphinyl, trifluoromethylsulphonyl, chlorodifluoromethoxy, chlorodifluoromethylthio or N-trifluoromethyl-N-dichlorofluoromethylthio-amino,

$R^6$ and $R^7$ represent hydrogen or fluorine and n represents 0 or 1,

with the proviso that one of the radicals $R^3$ to $R^7$ is a fluorine-containing radical or at least 3 of the radicals $R^3$ to $R^7$ represent fluorine and/or chlorine.

4. Process for the preparation of acyloxythiophene derivatives of the general formula (I)

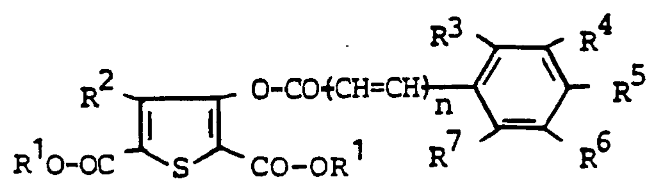

(I)

in which

$R^1$ represents alkyl with 1 to 5 carbon atoms, alkoxyalkyl or alkylthioalkyl with 1 to 5 carbon atoms per alkyl part, halogenoalkyl with 1 or 2 carbon atoms and up to 5 identical or different halogen atoms, cyanoalkyl with 1 to 5 carbon atoms in the alkyl part, trialkylsilylmethyl with 1 to 3 carbon atoms per alkyl part, alkenyl with 2 to 4 carbon atoms or alkinyl with 3 to 5 carbon atoms, $R^2$ represents alkyl with 1 to 4 carbon atoms, n represents 0 or 1,

$R^3$ to $R^5$ are identical or different and represent hydrogen, alkyl or alkoxy with 1 to 4 carbon atoms, halogenoalkyl with 1 or 2 carbon atoms and up to 5 identical or different halogen atoms, halogenoalkoxy, halogenoalkylthio, halogenoalkylsulphinyl or halogenoalkylsulphonyl with 1 or 2 carbon atoms and up to 5 identical or different halogen atoms per halogenoalkyl radical, N-halogenoalkyl-N-halogenoalkylthio-amino with 1 or 2 carbon atoms and up to 5 identical or different halogen atoms per halogenoalkyl radical, chlorine or fluorine and

$R^6$ and $R^7$ represent hydrogen, fluorine, chlorine or trifluoromethyl, with the proviso that one of the radicals $R^3$ to $R^7$ is a fluorine-containing radical or at least 3 of the radicals $R^3$ to $R^7$ represent fluorine and/or chlorine, characterised in that a carboxylic acid derivative of the general formula (II)

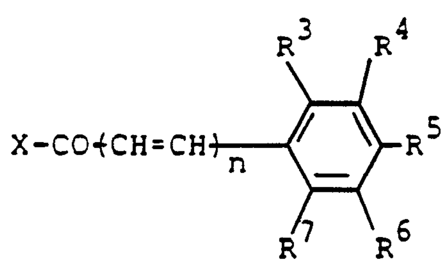

(II)

in which

X represents halogen, hydroxyl or the radical

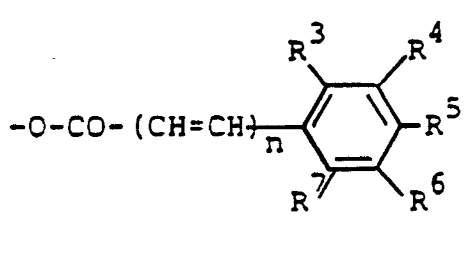

where, in the formulae,

n and $R^3$ to $R^7$ have the abovementioned meaning, is reacted with 3-hydroxy-thiophene derivatives of the general formula (III)

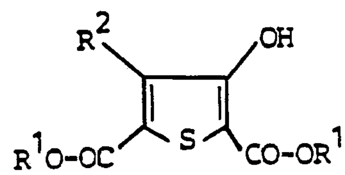

(III)

$R^1$ and $R^2$ have the abovementioned meaning, if appropriate in the presence of a solvent or diluent and if appropriate in the presence of an acid-binding agent or dehydrating agent.

5. Plant protection agents, characterized in that they contain at least one acyloxythiophene derivative of the formula (I) according to Claim 1.

5. Method of combating fungi and bacteria, characterized in that acyloxythiophene derivatives of the formula (I) according to Claim 1 are allowed to act on fungi or bacteria or their environment.

7. Use of acyloxythiophene derivatives of the formula (I) according to Claim 1 for combating fungi and bacteria.

8. Process for the preparation of plant protection agents, characterized in that acyloxythiophene derivatives of the formula (I) according to Claim 1 are mixed with extenders and/or surface-active agents.